# EUROPEAN PATENT SPECIFICATION

(11) **EP 3 067 074 B1**
(45) Date of publication and mention of the grant of the patent: **27.10.2021**
(21) Application number: 13897090.0
(22) Date of filing: 15.11.2013
(51) Int. Cl.: A61L 27/20, A61L 27/24, A61K 8/65, A61K 31/728, A61P 17/02, A61P 19/08, A61P 43/00, A61L 27/56, A61L 27/26

(54) **BIOMATERIAL HAVING ENHANCED RUBBER PROPERTIES THROUGH NATURAL CROSS-LINKAGE OF COLLAGEN AND HYALURONIC ACID, PREPARING METHOD THEREOF, AND USING METHOD THEREOF**
BIOMATERIAL MIT VERBESSERTEN KAUTSCHUKEIGENSCHAFTEN DURCH NATÜRLICHE VERNETZUNG VON KOLLAGEN UND HYALURONSÄURE, HERSTELLUNGSVERFAHREN DAFÜR UND VERWENDUNGSVERFAHREN DAFÜR
BIOMATÉRIAU AYANT DES PROPRIÉTÉS DE CAOUTCHOUC AMÉLIORÉES AU MOYEN D'UNE RÉTICULATION NATURELLE DE COLLAGÈNE ET D'ACIDE HYALURONIQUE, SON PROCÉDÉ DE PRÉPARATION ET SON PROCÉDÉ D'UTILISATION

(30) Priority: 08.11.2013 KR 20130135767
(43) Date of publication of application: 14.09.2016
(73) Proprietor: CELLONTECH Co., Ltd., Seoul, 04783 (KR)
(72) Inventor: YOO, Ji Chul, Namyangju-si Gyeonggi-do 472-869 (KR); YEO, Se Ken, Suwon-si Gyeonggi-do 443-270 (KR); LEE, Myoung Sung, Bucheon-si Gyeonggi-do 420-761 (KR); LEE, Jun Keun, Seoul 131-816 (KR); KO, Chang Kwon, Seoul 139-921 (KR); SUH, Dong Sam, Seoul 139-924 (KR); CHANG, Cheong Ho, Seoul 135-841 (KR)
(74) Representative: dompatent von Kreisler Selting Werner - Partnerschaft von Patent- und Rechtsanwälten mbB
(86) International application number: PCT/KR2013/010399
(87) International publication number: WO 2015/068884

(56) References cited:
- EP-A1- 1 935 438
- EP-A1- 2 851 096
- EP-A2- 2 514 445
- JP-B2- 5 289 772
- KR-A- 20120 084 189
- KR-B1- 100 846 311
- KR-B1- 101 047 510
- KR-B1- 101 279 812
- US-A1- 2003 100 739
- US-A1- 2013 129 835
- None

## Description

### Technical Field

The present invention relates to a biomaterial having enhanced rubber properties through natural cross-linking of collagen and hyaluronic acid, a method of preparing the same, and a method of using the same and, more particularly, to a novel material that exhibits rubber properties, which is prepared from collagen and hyaluronic acid at an optimal mixing ratio taking into consideration the molecular structural properties thereof under natural cross-linking conditions, rather than chemical or physical cross-linkage, thereby remarkably improving the quality and reliability of products, ultimately fulfilling various needs of consumers to thus increase marketability.

### Background Art

As known in the art, collagen and hyaluronic acid are currently widely useful as materials for application to the living body.

Collagen is a structural protein that constitutes the skin, string/tendons, blood vessels, bones, cartilage, spinal discs, etc. Collagen is widely utilized for tissue engineering, such as a bone graft material, a wound dressing, a cosmetic filler, and cell culture.

Also, hyaluronic acid is a carbohydrate that is incorporated in the skin, cartilage, eyes, etc., and is provided in the form of a product such as a joint lubricant, a cosmetic filler, an attachment inhibitor, etc.

However, when such materials that constitute the living body are used alone, they have poor physical strength and are easily degraded, and thus are cross-linked, simply mixed, or coupled with a synthetic material in order to serve as a biomaterial, thereby ensuring desired strength and degradability. The materials thus used may cause side effects in the course of biodegradation of any material other than *in-vivo* material, and are not degraded in the tissue regeneration step, and may rather function as an interfering material.

With the goal of solving such conventional problems, the patent that is described below is filed and laid open.

Such a conventional technique is directed to a medical bio-composite material containing collagen and hyaluronic acid, and to a method of preparing a composition by simply mixing collagen and hyaluronic acid and cross-linking the mixture with a chemical material.

However, the above technique may incur the following problems. Specifically, the conventional technique is problematic because the simple mixture of collagen and hyaluronic acid cannot be made into a desired formulation unless a chemical cross-linking material is used.

The conventional technique adopts a cross-linking process through simple mixing, especially a cross-linking process using a chemical material, thus making it impossible to completely exclude problems related to the safety of remaining materials, degrading materials or cross-linking materials. Furthermore, since the usable formulation may be provided only in a dry phase, limitations are imposed on the usage fields and use methods thereof.

Consequently, the method of the conventional technique is not suitable for industrial purposes and is neither simple nor safe.

### [Citation List]

(Patent Document 1) Korean Patent Application Publication No. 2013-0009651 (Title: Cell therapy product for cartilage damage comprising collagen, hyaluronic acid derivative and mammalian umbilical cord-derived stem cells). The patent application document KR 101 279 812 B1 discloses a composition for cartilage tissue repair being prepared by mixing a solution of collagen with a solution of hyaluronic acid.

### Disclosure

### Technical Problem

Therefore, the present invention has been made keeping in mind the problems encountered in the related art, a first object of the present invention is to provide a rubber-type biomaterial resulting from natural cross-linking of collagen and hyaluronic acid at a ratio of 2:1 to 7:1; a second object of the present invention is to provide the preparation of a material having properties of a rubber formulation under natural cross-linking conditions, rather than through chemical or physical cross-linking methods; a third object of the present invention is to provide a biomaterial that is not easily dissolved in water and has enhanced physical properties and degradability; a fourth object of the present invention is to provide a method of variously and widely using the biomaterial having such properties for a skin graft material, a wound dressing, a bone graft material, and a support for cell culture; a fifth object of the present invention is to provide a preparation method that is industrially simple and safe; and a sixth object of the present invention is to provide a biomaterial, a method of preparing the same, and a method of using the same, wherein the biomaterial is imparted with enhanced rubber properties through the natural cross-linking of collagen and hyaluronic acid, whereby the quality and reliability of a product may be considerably improved to thereby increase marketability to a consumer.

### Technical Solution

In order to accomplish the above objects, the present invention provides a biomaterial having enhanced rubber properties through natural cross-linking of collagen and hyaluronic acid, wherein a rubber-type biomaterial is prepared from collagen and hyaluronic acid at a ratio of 2:1 to 7:1 through natural cross-linking according to the appended claims.

In addition, the present invention provides a method of preparing a biomaterial having enhanced rubber properties through natural cross-linking of collagen and hyaluronic acid, suitable for preparation of a rubber-type biomaterial through natural cross-linking from collagen and hyaluronic acid at a ratio of 2:1 to 7:1 using syringe mixing, comprising:
providing a collagen solution which is formed under an acidic condition and a hyaluronic acid solution which is formed under a neutral condition;
and subjecting the collagen and hyaluronic acid to natural cross-linking at a ratio of 2:1 to 7:1, thus producing the biomaterial having rubber properties.

In addition, the present invention provides a method of preparing a biomaterial having enhanced rubber properties through natural cross-linking of collagen and hyaluronic acid, suitable for preparation of a rubber-type biomaterial through natural cross-linking from collagen and hyaluronic acid at a ratio of 2:1 to 7:1 using centrifugation, comprising:
providing a collagen solution which is formed under an acidic condition and a hyaluronic acid solution which is formed under a neutral condition;
mixing the collagen and hyaluronic acid at a ratio of 3:1; applying vigorous mixing and centrifugal force to a mixed solution, thus producing a lump; and removing the solution other than the lump, and concentrating and dewatering the lump using centrifugation or a kneader, thus obtaining the biomaterial in a rubber formulation.

In addition, the present invention provides a biomaterial having enhanced rubber properties through natural cross-linking of collagen and hyaluronic acid, comprising: using a rubber-type biomaterial, resulting from natural cross-linking of collagen and hyaluronic acid at a ratio of 2:1 to 7:1 according to the appended claims, for use as a cosmetic filler and a sealant by forming the rubber-type biomaterial into an injectable formulation placed in an injection container using a loading device.

In addition, the present invention provides a biomaterial having enhanced rubber properties through natural cross-linking of collagen and hyaluronic acid, comprising: using a rubber-type biomaterial, resulting from natural cross-linking of collagen and hyaluronic acid at a ratio of 2:1 to 7:1 according to the appended claims, for use as a cosmetic filler, a wound dressing and a coating material by forming the rubber-type biomaterial into a matrix formulation through lyophilization.

In addition, the present invention provides a biomaterial having enhanced rubber properties through natural cross-linking of collagen and hyaluronic acid, comprising: using a rubber-type biomaterial, resulting from natural cross-linking of collagen and hyaluronic acid at a ratio of 2:1 to 7:1 according to the appended claims, for use as a wound dressing, a bone graft material and a coating material by forming the rubber-type biomaterial into a layered porous matrix formulation through 3D printing.

In addition, the present invention provides a biomaterial having enhanced rubber properties through natural cross-linking of collagen and hyaluronic acid, comprising: using a rubber-type biomaterial, resulting from natural cross-linking of collagen and hyaluronic acid at a ratio of 2:1 to 7:1 according to the appended claims, for use as a cosmetic filler, a bone graft material, a wound dressing and a coating material by forming the rubber-type biomaterial into a formulation through mixing in a carrier application.

In addition, the present invention provides a biomaterial having enhanced rubber properties through natural cross-linking of collagen and hyaluronic acid, comprising: using a rubber-type biomaterial, resulting from natural cross-linking of collagen and hyaluronic acid at a ratio of 2:1 to 7:1 according to the appended claims, for use as a wound dressing, a dental material and a coating material by drying the rubber-type biomaterial using compression.

In addition, the present invention provides a biomaterial having enhanced rubber properties through natural cross-linking of collagen and hyaluronic acid, comprising: using a rubber-type biomaterial, resulting from natural cross-linking of collagen and hyaluronic acid at a ratio of 2:1 to 7:1 according to the appended claims, for use as a bone graft material, a wound dressing, a dental material, a filling material, a hemostatic material and a cell/tissue mixture by subjecting the rubber-type biomaterial to lyophilization and powdering using a grinder.

### Advantageous Effects

According to the present invention, a rubber-type biomaterial can be prepared from collagen and hyaluronic acid at a ratio of 2:1 to 7:1 through natural cross-linking.

Also, according to the present invention, the material, having properties of a rubber formulation, can be prepared under natural cross-linking conditions, rather than through chemical or physical cross-linking methods.

In particular, the biomaterial of the present invention is not easily dissolved in water, and is enhanced in physical properties and degradability.

Furthermore, the present invention can provide the method of variously and widely using the biomaterial having such properties for a skin graft material, a wound dressing, a bone graft material, and a support for cell culture.

Therefore, the present invention can provide a preparation method that is industrially simple and safe.

Thanks to the above effects, the quality and reliability of products can be significantly improved, thus increasing marketability to the consumer.

Preferred embodiments of the present invention for achieving the above effects are described in detail below with reference to the appended drawings.

### Description of Drawings

FIG. 1 is an electron microscope image illustrating collagen and hyaluronic acid, which are conventionally simply mixed;
FIGS. 2(a) and 2(b) are images illustrating a rubber formulation of collagen and hyaluronic acid according to the present invention;
FIG. 3 illustrates a formulation of collagen and hyaluronic acid, which are naturally cross-linked according to the present invention;
FIG. 4 is an electron microscope image illustrating the formulation of collagen and hyaluronic acid, which are naturally cross-linked according to the present invention;
FIG. 5 illustrates a formulation configured such that the biomaterial according to the present invention is placed in an injection container;
FIG. 6 illustrates a matrix formulation formed through lyophilization of the biomaterial according to the present invention;
FIG. 7 illustrates a matrix formulation formed through 3D printing of the biomaterial according to the present invention;
FIG. 8 illustrates a mixed formulation of the biomaterial according to the present invention in a carrier application;
FIG. 9 illustrates a sheet formulation formed through compression of the biomaterial according to the present invention;
FIG. 10 illustrates a powder formulation of the biomaterial according to the present invention using a grinder; and
FIGS. 11(a), 11(b) and 11(c) illustrate the formulation of the present invention using a kneader.

### Best Mode

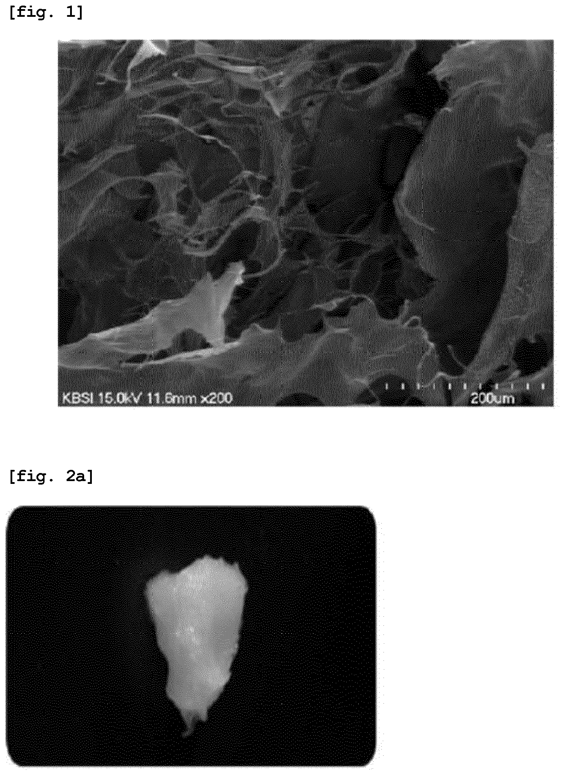

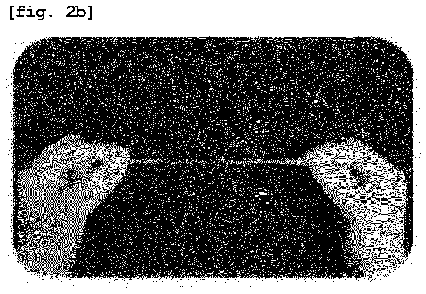

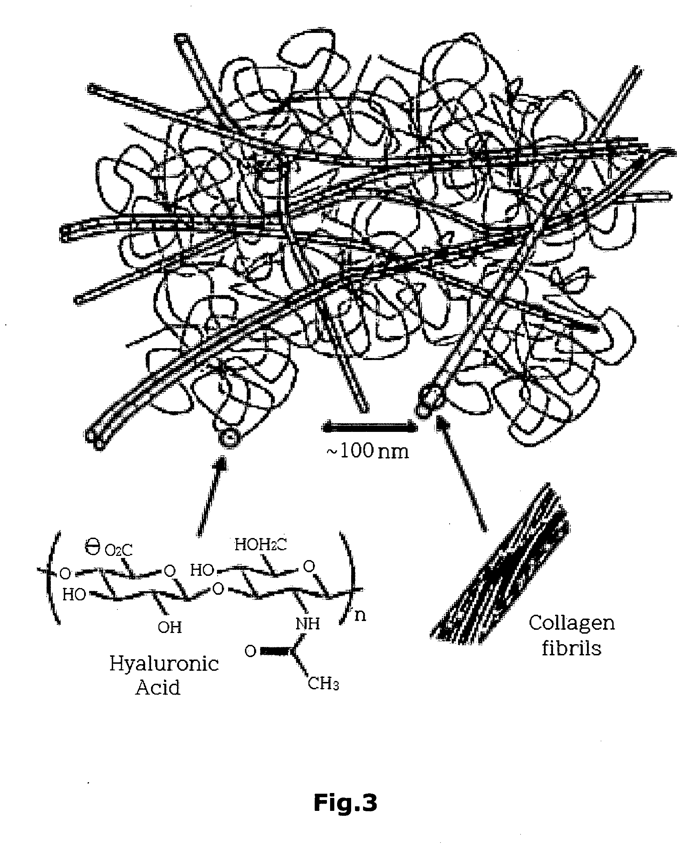

According to the present invention, a biomaterial having enhanced rubber properties through natural cross-linking of collagen and hyaluronic acid, a method of preparing the same, and a method of using the same are constructed as illustrated in FIGS. 2 to 11.

The present invention addresses a biomaterial having enhanced rubber properties through natural cross-linking of collagen and hyaluronic acid, wherein the rubber-type biomaterial is prepared from collagen and hyaluronic acid at a ratio of 2:1 to 7:1 through natural cross-linking according to the appended claims.

According to the present invention, collagen is a protein that structurally comprises three strands, in which glycine, proline, alanine, and hydroxyproline, in that order, are the abundant amino acid residues, glycine is an important amino acid for forming the three-stranded structure of collagen, the glycine residue is positively charged (+), and the positive charge (+) of glycine and the ions of proline and hydroxyproline residues form molecular bonds through hydrogen bonding to thus maintain a predetermined morphology. Hyaluronic acid is a carbohydrate, has a polysaccharide structure, and is a negatively charged (-) material containing a plurality of carboxyl groups. There is thus provided a biomaterial that is imparted with enhanced rubber properties through the natural cross-linking between collagen and hyaluronic acid.

Particularly in the present invention, the hyaluronic acid preferably has a molecular weight of 900 to 1,100 Kda.

Furthermore, the collagen solution and the hyaluronic acid solution, used in the present invention, are formed under an acidic condition and a neutral condition, respectively.

Below is a description of the biomaterial having enhanced rubber properties through natural cross-linking of collagen and hyaluronic acid, the method of preparing the same, and the method of using the same, according to the present invention.

In the present invention, a novel material that exhibits rubber properties is prepared from collagen and hyaluronic acid at an optimal mixing ratio taking into consideration the molecular structural properties thereof under natural cross-linking conditions, rather than chemical or physical cross-linkage.

According to a first embodiment of the present invention, the method of preparing a biomaterial having enhanced rubber properties through natural cross-linking of collagen and hyaluronic acid, suitable for the preparation of a rubber-type biomaterial through natural cross-linking from collagen and hyaluronic acid at a ratio of 2:1 to 7:1 using syringe mixing, includes providing a collagen solution which is formed under an acidic condition and a hyaluronic acid solution which is formed under a neutral condition; and subjecting the collagen and hyaluronic acid to natural cross-linking at a ratio of 2:1 to 7:1 under the condition that the concentration of each solution is 1%, thus producing the biomaterial having rubber properties.

According to a second embodiment of the present invention, the method of preparing a biomaterial having enhanced rubber properties through natural cross-linking of collagen and hyaluronic acid, suitable for the preparation of a rubber-type biomaterial through natural cross-linking from collagen and hyaluronic acid at a ratio of 2:1 to 7:1 using centrifugation, includes providing a collagen solution which is formed under an acidic condition and a hyaluronic acid solution which is formed under a neutral condition; mixing the collagen and hyaluronic acid at a ratio of 3:1 under the condition that the concentration of each solution is 1%; applying strong energy (centrifugal force) to the mixed solution, thus producing a lump; and removing the solution other than the lump, and concentrating and dewatering the lump using centrifugation or a kneader, thus obtaining the biomaterial in a rubber formulation.

The present invention addresses the following use method through the above preparation method.

According to a first embodiment of the present invention, a rubber-type biomaterial, resulting from natural cross-linking of collagen and hyaluronic acid at a ratio of 2:1 to 7:1, is used as a cosmetic filler and a sealant by forming the rubber-type biomaterial into an injectable formulation placed in an injection container using a loading device.

According to a second embodiment of the present invention, a rubber-type biomaterial, resulting from natural cross-linking of collagen and hyaluronic acid at a ratio of 2:1 to 7:1, is used as a cosmetic filler, a wound dressing and a coating material by forming the rubber-type biomaterial into a matrix formulation through lyophilization.

According to a third embodiment of the present invention, a rubber-type biomaterial, resulting from natural cross-linking of collagen and hyaluronic acid at a ratio of 2:1 to 7:1, is used as a wound dressing, a bone graft material, and a coating material by forming the rubber-type biomaterial into a layered porous matrix formulation through 3D printing.

According to a fourth embodiment of the present invention, a rubber-type biomaterial, resulting from natural cross-linking of collagen and hyaluronic acid at a ratio of 2:1 to 7:1, is used as a cosmetic filler, a bone graft material, a wound dressing, and a coating material by forming the rubber-type biomaterial into a formulation through mixing in a carrier application.

According to a fifth embodiment of the present invention, a biomaterial having enhanced rubber properties through natural cross-linking of collagen and hyaluronic acid includes using a rubber-type biomaterial, resulting from natural cross-linking of collagen and hyaluronic acid at a ratio of 2:1 to 7:1, is used as a wound dressing, a dental material, and a coating material by drying the rubber-type biomaterial using compression.

According to a sixth embodiment of the present invention, a biomaterial having enhanced rubber properties through natural cross-linking of collagen and hyaluronic acid includes: using a rubber-type biomaterial, resulting from natural cross-linking of collagen and hyaluronic acid at a ratio of 2:1 to 7:1, is used as a bone graft material, a wound dressing, a dental material, a filling material, a hemostatic material, and a cell/tissue mixture by subjecting the rubber-type biomaterial to lyophilization and powdering using a grinder.

Below is a detailed description of the present invention.

Useful in the present invention, collagen and hyaluronic acid are biocompatible materials, and are widely used as raw materials for manufacturing products to be applied to the living body. These materials are easily degraded and have somewhat poor physical properties when used alone in biological tissue.

However, such materials are interconnected with each other in tissue due to the inherent molecular structures and properties thereof and thus constitute and maintain the tissue and maintain life through biochemical reaction.

In order to develop a biomaterial, the present invention is ideally intended to provide a material very similar to the corresponding tissue to thus induce tissue regeneration, which is receiving a particularly high amount of attention when the material for use in graft treatment has to possess functionality. In the case of bone, autogenous bone, which is the ideal treatment material, is grafted. This is because the most preferable effects may be exhibited when autogenous bone is grafted. However, the supply of actual graft materials is limited, and thus, xenogeneic bone or alloplastic bone is mainly used. Alloplastic bone is made by synthesizing chemical materials, and is used by making a component, such as hydroxyapatite, which is present in the bone. Similar attempts have been made to provide materials similar to tissue so as to treat and regenerate the tissue.

Soft tissue is configured such that a protein component coexists with a carbohydrate component. In particular, the typical material of protein is collagen, and collagen is a structural protein that constitutes 80% or more of the protein present in biological tissue. The typical material of carbohydrate is hyaluronic acid. Hyaluronic acid exists in the cartilage, skin, etc., and is principally introduced in product form.

In particular, organic material that is present in cartilage comprises protein and carbohydrate at a ratio of 3:1. Also, the spinal disc is composed of nucleus pulposus and annulus fibrosus, the annulus fibrosus including protein and carbohydrate at a ratio of 3:1.

The ratio of protein to carbohydrate for use in constituting the tissue has been experimentally observed in order to make the ideal material very similar to tissue. For this approach, products are made using the materials from functional and economic points of view, and materials that may be applied and in which the morphology thereof may be maintained in water through physical or chemical cross-linking, have been currently developed.

The present invention pertains to a method of producing a safe material on a large scale, which is not dissolved in water, retains its degradability, and is biocompatible for tissue regeneration, through molecular structural bonding based on the inherent properties of the biocompatible materials, rather than through artificial physical or chemical cross-linking methods. The materials used therefor are representatively exemplified by collagen and hyaluronic acid, which are mixed at various ratios so as to yield rubber formulations. Collagen is a protein that structurally comprises three strands, in which glycine, proline, alanine, and hydroxyproline, in that order, are the abundant amino acid residues. Glycine, which is an important amino acid for forming the three-stranded structure of collagen, and glycine residue is positively charged (+). The positive charge (+) of glycine and the ions of proline and hydroxyproline residues form molecular bonds through hydrogen bonding to thus maintain a predetermined morphology. Hyaluronic acid is a carbohydrate, has a polysaccharide structure, and is a negatively charged (-) material containing a plurality of carboxyl groups. Thus, collagen cations and hyaluronic acid anions are coupled through hydrogen bonding, whereby the surface of collagen, which forms a fibrous shape, is covered with hyaluronic acid. In particular, based on the results of degradation using lyases such as collagenase and hyaluronidase, the materials used in the invention exhibits degradation performance more sensitive to hyaluronidase than to collagenase, and thus a morphology showing the coupled structure thereof may be expected. Also, the rubber-like properties are deemed to result from the application of a predetermined quantity of energy between two materials to thus induce coupling of the two materials, rather than simply mixing two materials.

Even when a cross-linking material is not added, the biomaterial obtained thus is not dissolved in a liquid, but maintains its morphology, and exhibits surface adhesion or softness that stretches.

The biomaterial having rubber-like properties produced according to the present invention is configured such that two materials are imparted with a connection structure through natural bonding as shown by electron microscopy, quite unlike the structure resulting from simple mixing of collagen and hyaluronic acid.

This biomaterial may be placed in an injection container and may be injected *in vivo* without surgical incision, or may be provided in a matrix through lyophilization and may thus be used to retain morphology. In particular, the lyophilized form has high strength and is thus favorable when the morphology of a tissue is required to be maintained for a predetermined period of time.

The rubber formulation of collagen and hyaluronic acid is configured as follows.

In the present invention, as illustrated in FIG. 2, the naturally cross-linked material, exhibiting softness like rubber, is prepared from collagen and hyaluronic acid.

When the ratio of collagen to hyaluronic acid falls in the range of 2:1 to 7:1, a rubber formulation is produced. The optimal ratio is 3:1.

In the present invention, collagen is atelocollagen comprising three strands, and the molecular weight of hyaluronic acid is 1,000 Kda. When the concentration of each of the dissolved materials is 10% (w/v) or more, it is impossible to form the formulation. Furthermore, the collagen solution and the hyaluronic acid solution require an acidic condition and a neutral condition, respectively.

The formulation of collagen and hyaluronic acid may be provided in the form of an injectable formulation by being loaded in an injection container, a matrix formulation through lyophilization, a sheet formulation through compression, or a powder formulation using a grinder, and may thus be variously applied.

Also, the biomaterial of the invention may be provided in the form of a layered porous matrix through 3D printing, and may thus have a desired structure. This formulation may be commercially available in putty form, or may be used as a matrix in a dry phase, through lyophilization.

The rubber formulation may be used in a carrier application, by incorporating an inorganic material, cell, tissue, growth factor, drug, protein, DNA, etc. therein.

The method of preparing the biomaterial having rubber properties from collagen and hyaluronic acid according to the present invention and the method of using the same are described below.

According to the present invention, the biomaterial, which is not dissolved in water, is prepared through natural cross-linking, rather than through physical or chemical cross-linking, the natural cross-linking being induced based on the inherent molecular properties of collagen and hyaluronic acid at a predetermined mixing ratio. The prepared biomaterial is neither easily dissolved in water nor easily degraded and has rubber-like properties, and may thus be applied in a variety of fields. In particular, a method of using the biomaterial prepared thus as a wound dressing, a bone graft material, a cosmetic filler, and a material for culturing cells and tissues is provided, and the preparation method thereof, which is industrially simple and safe, is provided.

A better understanding of the present invention is given through the following examples.

### (Example 1)

Preparation of Rubber Formulation Material through Natural Cross-linking of Collagen and Hyaluronic acid: Syringe Mixing
1. Object: Conditions for formulation using collagen and hyaluronic acid are checked.
2. Method: A collagen solution and a hyaluronic acid solution, each having a concentration of 1% (w/v), are prepared. Individual raw materials are mixed at a ratio of 1:2 to 9:1 (collagen:hyaluronic acid, whereby a ratio of 2:1 to 7:1 is according to the invention). While individual solutions loaded in syringes are combined from respective directions via a connector, the production of the rubber formulation is checked. The hyaluronic acid used has a molecular weight of 1,000 Kda.
3. Result: The naturally cross-linked materials having rubber properties were produced from individual raw materials having a concentration of 1% through mixing at a ratio of 2:1 to 7:1. The optimal mixing ratio was 3:1. At this ratio, the formulation having the highest viscosity was produced within a short time. The formulation having rubber properties could not be produced at a ratio higher or lower than the above ratio.

### (Example 2)

Preparation of Rubber Formulation through Natural Cross-linking of Collagen and Hyaluronic acid: Centrifugation
1. Object: A method for preparing collagen and hyaluronic acid on a large scale is checked.
2. Method: A collagen solution and a hyaluronic acid solution, each having a concentration of 1% (w/v), are prepared. Individual raw materials are mixed at a ratio of 3:1 (collagen:hyaluronic acid). When impact is applied to the mixed solution using strong energy, a lump is produced. The solution, other than the lump, is removed, and the lump is concentrated and dewatered using centrifugation, yielding a rubber formulation material.

### (Example 3)

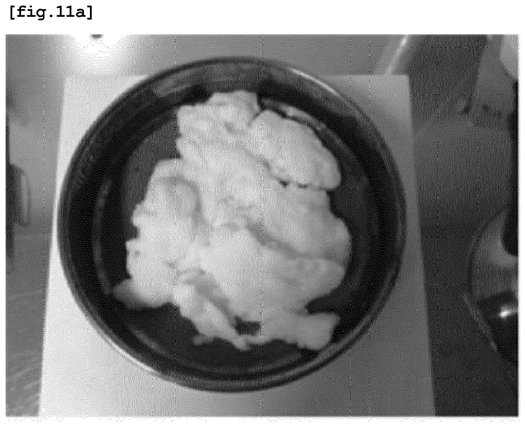

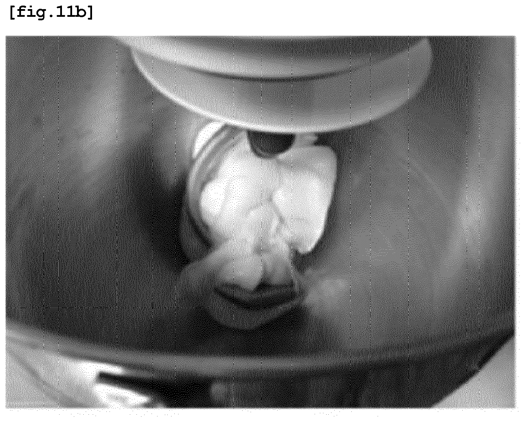

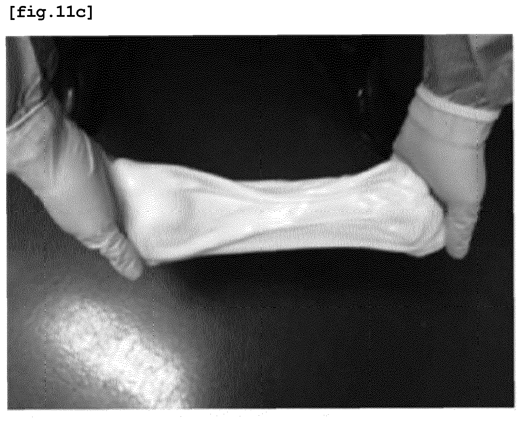

Preparation of Rubber Formulation through Natural Cross-linking of Collagen and Hyaluronic acid: Kneader
1. Object: A method for preparing collagen and hyaluronic acid on a large scale is checked.
2. Method: Collagen and hyaluronic acid are mixed at a ratio of 3:1 using a mixer, allowed to stratify, sieved to remove water, and kneaded to increase softness. The steps for preparing the formulation are shown in FIGS. 11(a), 11(b) and 11(c).

### (Example 4)

Measurement of Properties of Naturally Cross-linked Material of Collagen and Hyaluronic acid
1. Object: Adhesion of a naturally cross-linked material according to the present invention is analyzed based on maximum stress and yield strength.
2. Method: Each of the naturally cross-linked materials, obtained by mixing collagen and hyaluronic acid at a mixing ratio ranging from 2:1 to 7:1, is placed on a sampling vessel, and a thin film is attached thereto, after which the film is bent and connected to a load cell, and the table is moved downward at a preset speed using a predetermined force, and adhesion is measured while the film is separated. The maximum stress and yield strength thereof are measured using a Rhometer (CR-500DX, Sun scientific rheometer). The Tack rheology testing conditions were applied, the table speed was 300 mm/min, and the maximum stress was 2 kg.
3. Result: The naturally cross-linked rubber formulations produced under individual mixing conditions did not show any difference within the error range. The results are given as follows.

**[Table 1]**

| Mixing ratio (Collagen:Hyaluronic acid) | Maximum Stress (N) | Yield Strength (N) |
|---|---|---|
| 2:1 | 2.75±0.20 | 2.74±0.20 |
| 3:1 | 2.95±0.03 | 2.94±0.04 |
| 5:1 | 3.07±0.04 | 3.05±0.03 |
| 7:1 | 3.17±0.33 | 3.17±0.33 |

### (Example 5)

Electron microscopy of Naturally Cross-linked Rubber Formulation of Collagen and Hyaluronic acid
1. Object: The structure of a naturally cross-linked material according to the present invention is observed using a scanning electron microscope (SEM) to evaluate the stretching properties thereof.
2. Method: The morphologies of a formulation obtained through simple mixing and a formulation obtained through application of energy, using collagen and hyaluronic acid at a ratio of 3:1, are observed using FE-SEM. To this end, a HITACHI/S-4700 was used at a resolution of 1.5 nm (15 kV) and 2.5 nm (1 kV) in the magnification range from 25X to 500,000X.
3. Result: In the formulation obtained through simple mixing, individual faces and lines of hyaluronic acid and collagen are observed in combined form, but in the naturally linked rubber formulation, a large number of connected portions for connecting faces and lines are observed, thus exhibiting stretching properties.

### (Example 6)

Test of Degradability of Naturally Cross-linked Material of Collagen and Hyaluronic acid
1. Object: The extent of degradation of the naturally cross-linked material according to the present invention is evaluated.
2. Method: The extent of degradation of the material according to the present invention is measured using lyases and saline and the degradability and stability of the formulation are measured through hydrothermal treatment. As such, a collagen matrix product, for example, Terrafoam, was used as a control. Examples of the lyases include collagenase (Roche) and hyaluronase (H-lase, Kuhnil Pharmaceutical), respective concentrations of which were 15 mU/mL and 15 IU/mL. The material was observed for 7 days and the reaction thereof was checked by allowing the material to stand at 37°C. When using saline, the material was observed under the condition that it was allowed to stand at room temperature, and hydrothermal treatment was performed using purified water at 50°C for 8 hr.
3. Results
A) Degradation rate/remainder after lyase treatment

**[Table 2]**

| Collagen-Hyaluronic acid Formulation | | Lyase treatment period | | |
|---|---|---|---|---|
| | | 1 day | 3 days | 7 days |
| Remainder | Collagenase treatment | 127% | 88% | 35% |
| | Hyaluronase treatment | 117% | 27% | 0 |

| | | | | |
|---|---|---|---|---|
| *The collagen matrix used as the control was degraded within one day by collagenase and the morphology thereof was maintained for 7 days when using hyaluronase. | | | | |

B) The volume of the material was increased by an average of 135% in saline on the first day, and the morphology thereof was maintained for 7 days. During the long-term testing, the morphology thereof was maintained for three months or more.
C) Based on the results of observing the formulation of the present invention through hydrothermal treatment for 8 hr, the volume thereof was maintained at 80%.

### (Example 7)

Formation of Naturally Cross-linked Collagen-hyaluronic acid Formulation

### 1) Formulation loaded in injection container

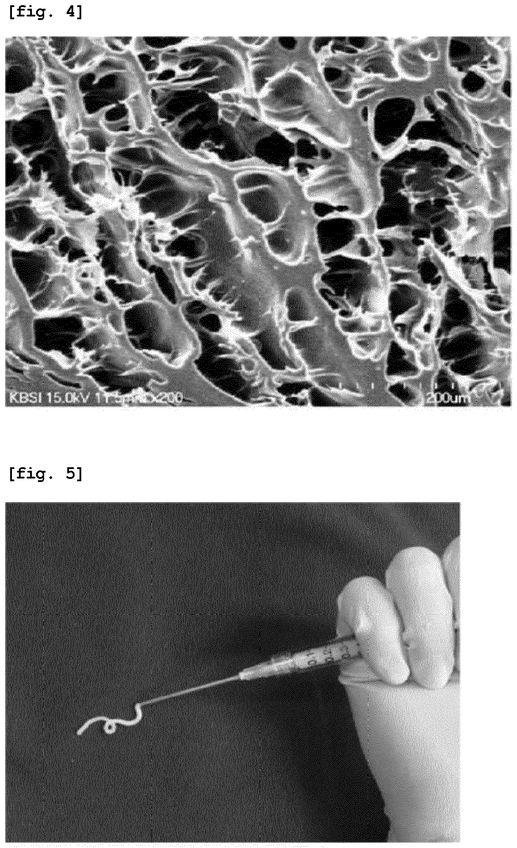

The material of the present invention was placed in an injection container using a loading device and was made into an injectable formulation, suitable for use as a cosmetic filler and a sealant, as illustrated in FIG. 5.

### 2) Lyophilized Formulation

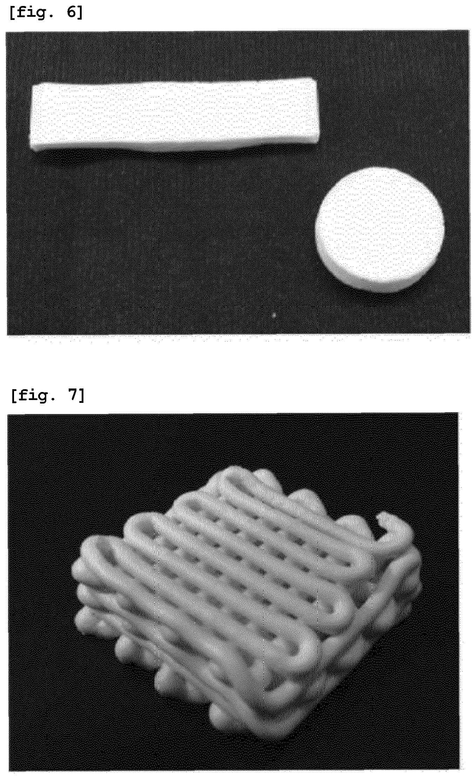

The material of the present invention was made into a matrix formulation through lyophilization, suitable for use as a cosmetic filler, a wound dressing and a coating material, as illustrated in FIG. 6.

### 3) 3D Printing Formulation

The material of the present invention was made into a layered porous matrix formulation through 3D printing, suitable for use as a wound dressing, a bone graft material, and a coating material, as illustrated in FIG. 7.

### 4) Mixed Formulation in Carrier application

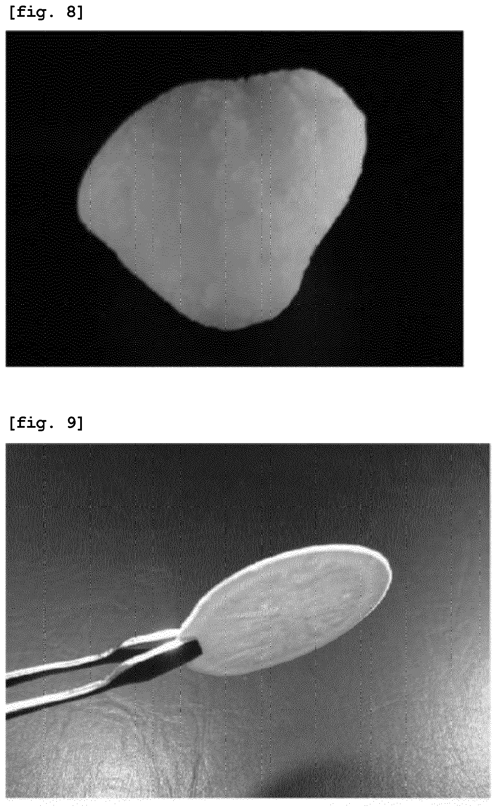

The material of the present invention was mixed with an additional component, for example, TCP, which is a bone replacement, and then made into a formulation suitable for use as a cosmetic filler, a bone graft material, a wound dressing and a coating material, as illustrated in FIG. 8.

### 5) Sheet Formulation

The material of the present invention was lyophilized using a compression process and made into a formulation suitable for use as a wound dressing, a dental material and a coating material, as illustrated in FIG. 9.

### 6) Powder Formulation

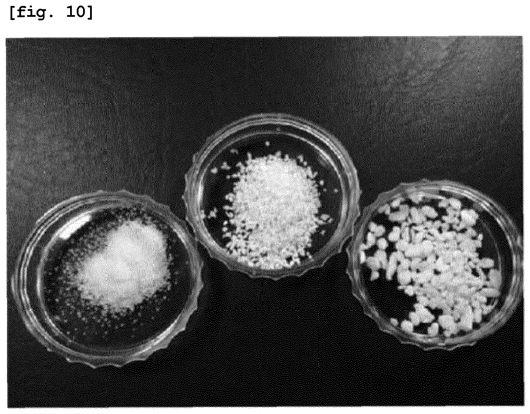

The material of the present invention was lyophilized, powdered using a grinder, and made into a formulation suitable for use as a bone graft material, a wound dressing, a dental material, a filling material, a hemostatic material, and a cell/tissue mixture, as illustrated in FIG. 10.

### Industrial Applicability

The industrial applicability of the present invention, regarding the biomaterial having enhanced rubber properties through natural cross-linking of collagen and hyaluronic acid, the method of preparing the same, and the method of using the same, has been actually realized with consistent, repeatable results. In particular, the present invention is carried out in this way to thereby promote the technical development in order to contribute to industrial development.

## Claims

1. A biomaterial having enhanced rubber properties through natural cross-linking of collagen and hyaluronic acid, wherein a rubber-type biomaterial is prepared from collagen and hyaluronic acid at a ratio of 2:1 to 7:1 through natural cross-linking; and
wherein a collagen solution is formed under an acidic condition and a hyaluronic acid solution is formed under a neutral condition.

2. The biomaterial of claim 1, wherein the hyaluronic acid has a molecular weight of 900 to 1,100 Kda.

3. A method of preparing a biomaterial having enhanced rubber properties through natural cross-linking of collagen and hyaluronic acid, suitable for preparation of a rubber-type biomaterial through natural cross-linking from collagen and hyaluronic acid at a ratio of 2:1 to 7:1
a) using syringe mixing, comprising:
providing a collagen solution which is formed under an acidic condition and a hyaluronic acid solution which is formed under a neutral condition ; and
subjecting the collagen and hyaluronic acid to natural cross-linking at a ratio of 2:1 to 7:1, thus producing the biomaterial having rubber properties; or
b) using centrifugation, comprising:
providing a collagen solution which is formed under an acidic condition and a hyaluronic acid solution which is formed under a neutral condition;
mixing the collagen and hyaluronic acid at a ratio of 3:1;
applying vigorous mixing and centrifugal force to a mixed solution, thus producing a lump; and
removing the solution other than the lump, and concentrating and dewatering the lump using centrifugation or a kneader, thus obtaining the biomaterial in a rubber formulation.

4. A biomaterial according to any one of claim 1 or 2 for use:
a) as a cosmetic filler and a sealant by forming the rubber-type biomaterial into an injectable formulation placed in an injection container using a loading device; or
b) as a cosmetic filler, and a coating material by forming the rubber-type biomaterial into a matrix formulation through lyophilization; or
c) as a coating material by forming the rubber-type biomaterial into a layered porous matrix formulation through 3D printing; or
d) as a cosmetic filler, and a coating material by forming the rubber-type biomaterial into a formulation through mixing in a carrier application; or
e) as a dental material and a coating material by drying the rubber-type biomaterial using compression; or
f) as a dental material, a filling material, and a cell/tissue mixture by subjecting the rubber-type biomaterial to lyophilization and powdering using a grinder.

## Patentansprüche

1. Biomaterial, das durch natürliche Vernetzung von Collagen und Hyaluronsäure verstärkte Kautschukeigenschaften aufweist, wobei ein Biomaterial des Kautschuktyps durch natürliche Vernetzung aus Collagen und Hyaluronsäure in einem Verhältnis von 2:1 bis 7:1 hergestellt ist; und wobei unter sauren Bedingungen eine Collagenlösung gebildet wird und unter neutralen Bedingungen eine Hyaluronsäurelösung gebildet wird.

2. Biomaterial gemäß Anspruch 1, wobei die Hyaluronsäure ein Molekulargewicht von 900 bis 1100 kDa aufweist.

3. Verfahren zur Herstellung eines Biomaterials, das durch natürliche Vernetzung von Collagen und Hyaluronsäure verstärkte Kautschukeigenschaften aufweist und für die Herstellung eines Biomaterials des Kautschuktyps durch natürliche Vernetzung aus Collagen und Hyaluronsäure in einem Verhältnis von 2:1 bis 7:1 geeignet ist:
a) unter Verwendung von Spritzenmischen, umfassend:
Bereitstellen einer Collagenlösung, die unter sauren Bedingungen gebildet wird, und einer Hyaluronsäurelösung, die unter neutralen Bedingungen gebildet wird; und
Durchführen einer natürlichen Vernetzung mit dem Collagen und der Hyaluronsäure in einem Verhältnis von 2:1 bis 7:1 und damit Herstellen des Biomaterials, das Kautschukeigenschaften aufweist; oder
b) unter Verwendung von Zentrifugation, umfassend:
Bereitstellen einer Collagenlösung, die unter sauren Bedingungen gebildet wird, und einer Hyaluronsäurelösung, die unter neutralen Bedingungen gebildet wird;
Mischen des Collagens und der Hyaluronsäure in einem Verhältnis von 3:1;
Anwenden von kräftigem Mischen und einer Zentrifugalkraft auf die gemischte Lösung, wobei ein Klumpen entsteht; und
Entfernen der Lösung außer dem Klumpen und Einengen und Entwässern des Klumpens unter Verwendung von Zentrifugation oder eines Kneters, wobei man das Biomaterial in einer Kautschukzubereitung erhält.

4. Biomaterial gemäß einem der Ansprüche 1 oder 2 zur Verwendung:
a) als kosmetischer Füllstoff und Dichtungsmittel durch Bilden einer injizierbaren Zubereitung aus dem Biomaterial des Kautschuktyps, die mit Hilfe einer Füllvorrichtung in einen Injektionsbehälter eingebracht wird; oder
b) als kosmetischer Füllstoff und Beschichtungsmaterial durch Bilden einer Matrixzubereitung durch Lyophilisierung aus dem Biomaterial des Kautschuktyps; oder
c) als Beschichtungsmaterial durch Bilden einer geschichteten porösen Matrixzubereitung durch 3D-Druck aus dem Biomaterial des Kautschuktyps; oder
d) als kosmetischer Füllstoff und Beschichtungsmaterial durch Bilden einer Zubereitung aus dem Biomaterial des Kautschuktyps durch Mischen in einer Trägeranwendung; oder
e) als Dentalmaterial und Beschichtungsmaterial durch Trocknen des Biomaterials des Kautschuktyps mit Hilfe von Kompression; oder
f) als Dentalmaterial, Füllstoff und ein Zell-/Gewebe-Gemisch durch Lyophilisieren des Biomaterials des Kautschuktyps und Pulverisieren unter Verwendung einer Schleifmaschine.

## Revendications

1. Biomatériau ayant des propriétés de caoutchouc augmentées par réticulation naturelle de collagène et d'acide hyaluronique, dans lequel un biomatériau de type caoutchouc est préparé à partir de collagène et d'acide hyaluronique dans un rapport de 2 : 1 à 7 : 1 par réticulation naturelle, et
dans lequel une solution de collagène est formée dans des conditions acidiques, et une solution d'acide hyaluronique est formée dans des conditions neutres.

2. Biomatériau selon la revendication 1, dans lequel l'acide hyaluronique présente un poids moléculaire de 900 à 1100 kDa.

3. Procédé pour préparer un biomatériau ayant des propriétés de caoutchouc augmentées par réticulation naturelle de collagène et d'acide hyaluronique, adapté pour préparer un biomatériau de type caoutchouc par réticulation naturelle à partir de collagène et d'acide hyaluronique dans un rapport de 2 : 1 à 7 : 1,
a) en utilisant le mélange dans une seringue, comprenant les étapes consistant à :
procurer une solution de collagène qui est formée dans des conditions acidiques, et une solution d'acide hyaluronique, qui est formée dans des conditions neutres, et
soumettre le collagène et l'acide hyaluronique à une réticulation naturelle dans un rapport de 2 : 1 à 7 : 1, ainsi produisant le biomatériau ayant des propriétés de caoutchouc, ou
b) en utilisant la centrifugation, comprenant les étapes consistant à :
procurer une solution de collagène qui est formée dans des conditions acidiques, et une solution d'acide hyaluronique, qui est formée dans des conditions neutres,
mélanger le collagène et l'acide hyaluronique dans un rapport de 3 : 1,
appliquer un mélange vigoureux et une force centrifuge à la solution mixte, ainsi produisant un morceau, et
éliminer la solution outre le morceau, et concentrer et déshydrater ledit morceau en utilisant la centrifugation ou un pétrin, obtenant ainsi ledit biomatériau dans une formulation de caoutchouc.

4. Biomatériau selon l'une des revendications 1 ou 2 à utiliser :
a) comme charge cosmétique et matériau d'étanchéité en formant le biomatériau de type caoutchouc en une formulation injectable placée dans un récipient d'injection en utilisant un dispositif de charge, ou
b) comme charge cosmétique et matériau de revêtement en formant le biomatériau de type caoutchouc en une formulation de matrice par lyophilisation, ou
c) comme matériau de revêtement en formant le biomatériau de type caoutchouc en une formulation de matrice poreuse en couches par imprimerie tridimensionnelle, ou
d) comme charge cosmétique et matériau de revêtement en formant le biomatériau de type caoutchouc en une formulation par mélange dans une application de véhicule, ou
e) comme matériau dental et matériau de revêtement par séchage du biomatériau de type caoutchouc en utilisant une compression, ou
f) comme matériau dental et matériau de charge et mélange de cellule/tissu en soumettant le biomatériau de type caoutchouc à la lyophilisation et pulvérisation en utilisant un broyeur.
